# EUROPEAN PATENT SPECIFICATION

(11) **EP 2 234 951 B1**
(45) Date of publication and mention of the grant of the patent: **19.10.2016**
(21) Application number: 08863888.7
(22) Date of filing: 22.12.2008
(51) Int. Cl.: C07C 37/74, C07C 39/04, C07C 37/68

(54) **PURIFICATION OF PHENOL**
AUFREINIGUNG VON PHENOL
PURIFICATION DE PHÉNOL

(30) Priority: 20.12.2007 EP 07150224
(43) Date of publication of application: 06.10.2010
(73) Proprietor: Borealis Technology OY, 06101 Porvoo (FI)
(72) Inventor: PUROLA, Veli-Matti, FIN-06650 Hamari (FI)
(74) Representative: Campbell, Neil Boyd
(86) International application number: PCT/EP2008/011005
(87) International publication number: WO 2009/080338

(56) References cited:
- WO-A-88/03524
- FR-A- 1 206 743
- US-A- 5 131 984

## Description

### Background of the Invention

### Field of the Invention

The present invention concerns a process for purifying a phenolic mixture containing phenol, water and at least one further organic component.

### Description of Related Art

Phenol is commonly manufactured through a cumene procedure, wherein cumene is oxidized to cumene hydroperoxide (CHP) and the resulting oxidation product mixture is concentrated and subjected to a cleavage reaction. Subsequently, the cleavage product mixture is conducted to a distillation section, wherein the main products of the cleavage reaction, i.e. phenol and acetone, are first separated and then purified through a series of distillation steps or other purification steps. The crude phenol resulting from the separation of phenol and acetone contains several by-products and impurities, such as α-methyl styrene (AMS), acetophenone residues, mesityl oxide, 2-methylbenzofurane, cyclohexanol, phenyl dimethyl carbinol and other organic components, e.g. carbonyls, including ketones, and dimers. Because of the differences in properties and reactivities of these components, some extensive purification procedures may be required to reach a sufficient phenol product quality.

In addition to the commonly used purification by conventional distillation, the prior art includes a combination of a distillation step and a resin treatment step, as presented in EP publications 1,395,540 and 1,727,779, wherein the phenol mixture to be purified is subjected to a resin treatment with an acidic resin, whereby higher boiling compounds, such as dimers, are formed. These higher boiling compounds are then separated from the product phenol through distillation.

The use of a resin treatment causes some impurities to react with phenol and release water. This causes the water concentration to increase downstream from the resin bed reactor. Although the phenol product is not required to be completely dry, limiting the amount of water in the phenol product is still advantageous.

US 5 131 984 describes a phenol purification process in which overheads from a phenol distillation column are transferred to a condensor and in which the condensate is returned to the phenol distillation column. A preferred option is that the condensor is a steam generating condensor, thereby condensing a major portion of said vapors whilst vaporising water and stripping acids from the condensor. A consequence of introducing water into the process is that there is an inevitable increase in the water content of the phenol stream. One aspect of the present invention is to obtain phenol with low water content.

FR 1 206 743 A discloses a phenol purification process and apparatus in which a phenol distillation column is connected in series to a first and second condensor. There is, however, no recycle from the second condensor. This has important consequences for the separation of phenol and light impurities. Additionally, the present inventors have determined that the absence of a recycle between the first and second steps reduces the phenol product yield.

After purification of phenol by conventional distillation or steam distillation, or by a resin treatment or another type of catalytic purification, a final stage of phenol purification is generally performed in a final phenol purification tower. Typically, the product is drawn as a side stream from the top section of the tower. The quality of the product is typically improved by increasing the purge streams, such as the bottom and condensate outlets, i.e. by changing the material balance of the distillation tower. The achieved product quality is a result of the separation performance of the purification tower, which is effected by, for example, a number of separation stages, the reboiler duty, the reflux, the operating pressure and the bottom and distillate streams. The vapor-liquid equilibrium limits the separation of impurities and, thus, the purge rates may have to be multiplied to improve the phenol product quality.

The need thus exists for a more effective and economical process for reducing the level of impurities and by-products, together with the level of water, to obtain a desired phenol product quality.

### Summary of the Invention

It is an aim of the present invention to provide an efficient phenol purification process.

Particularly, it is an aim of the present invention to provide a phenol purification process achieving a pure phenol product with a sufficiently low concentration of impurities and a lowered water-content.

It is a further aim of the invention to provide a phenol purification process with limited phenol recycle streams.

These and other objects, together with the advantages thereof over known processes, are achieved by the present invention, as hereinafter described and claimed.

The present invention concerns a process for purifying a phenolic mixture, which process comprises
- as a first step, providing a phenolic feed containing phenol, water and at least one further organic or acidic component, and
- as a second step, separating the phenolic feed by distillation into a base fraction, an intermediate fraction and a first overhead stream,
- conducting the first overhead stream through at least one condenser to an condensate drum 10 in which at least one first condensate and at least one first uncondensed fraction are formed,
- recycling at least a portion of the first condensate to the distillation;
- conducting the first uncondensed fraction to a second condenser wherein a second condensed fraction and a second uncondesned fraction are formed;
- removing at least a portion of the second uncondensed fraction;
- recycling at least a portion of the second condensed fraction to the condensate drum 10,
- removing at least a portion of said first and/or second condensed fraction.

The present invention also concerns an apparatus for purifying a phenolic mixture as claimed herein.

Considerable advantages are obtained by means of the invention. Thus, the present invention provides a process for effectively separating by-products, impurities and water from a phenol product without loosing significant amounts of product with the recycle streams. In particular, the use of two condensers and the condensate drum, with the recycle of the second condensate from the second condenser to the condensate drum maximises purification. The temperature within the drum encourages any light impurities which may have become incorporated within the first or second condensers to return to the second condenser where they are removed as an uncondensed overhead fraction. The amount of light materials actually recycled to the column is therefore minimised.

Next, the invention will be described more closely with reference to the attached drawing and a detailed description.

### Brief Description of the Drawing

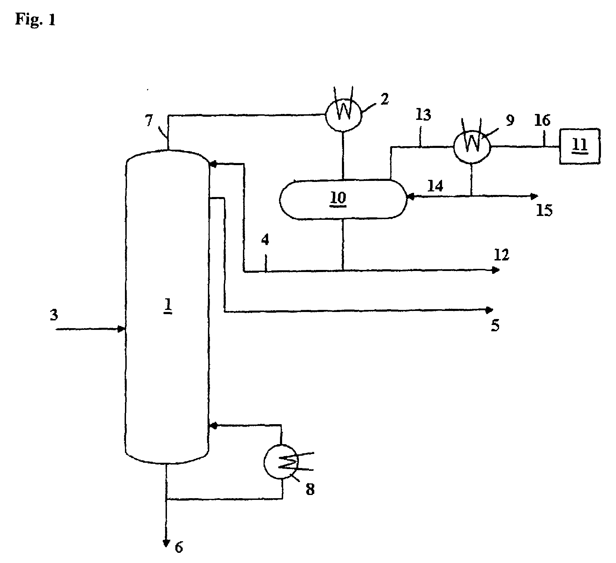

Figure 1 is a schematic representation of a preferred embodiment of the present invention.

### Detailed Description of the Preferred Embodiments of the Invention

The present invention concerns a process for purifying a phenolic mixture, which process comprises providing a phenolic feed containing phenol, water and at least one further organic or acidic component, and separating the phenolic feed by distillation into a base fraction, an intermediate fraction and a first overhead stream.

Particularly, the present invention concerns a phenol purification process comprising conducting the first overhead stream through at least one condenser and into an condensate drum, wherein at least one first condensate and corresponding uncondensed fraction(s) are formed, and recycling at least a portion of the first condensate(s) as reflux to the distillation. Whilst it is within the scope of the invention for all the condensate in drum 10 to be recycled, it is preferred if only a portion of it is recycled, e.g. at least 50 wt%, such as at least 70 wt%, or at least 90 wt%, even up to 95 wt% is recycled. It is preferred therefore if at least a portion of the condensate within drum 10 is removed.

The term "distillation" is intended to mean a method of separating chemical components based on the differences in their volatilities in a boiling liquid mixture. The mixture being distilled is separated into a overhead stream containing "light" or "low-boiling" components, i.e., components that vaporize under the conditions prevailing during the distillation, and an unevaporated fraction, or a base fraction, containing "heavy" or "high-boiling" components, i.e., components that remain in liquid form under the conditions prevailing during the distillation.

Likewise, a mixture conducted to a condenser is separated into a "condensate", containing the heavy components, and an "uncondensed fraction" (or an uncondensed vapor), containing the light components and noncodensable inert gases.

The identities of the compounds included in the heavy components and the identities of the compounds included in the light components depends on the prevailing conditions in the distillation column or in the condenser, such as the number of equilibrium stages, the temperature and the pressure.

The term "fractional distillation" is intended to mean a particular form of distillation, wherein a mixture is separated into its components or fractions by their volatilities by subjecting them to repeated vaporization-condensation cycles within a fractionation column.

A "column plate" or a "column tray" is a zone of a distillation column, in which two phases, such as the liquid and vapor phases of a substance, establish equilibrium with each other. Typically column trays do not reach a complete theoretical vapor-liquid equilibrium. The performance of some separation processes depends on having a series of equilibrium stages and is enhanced by providing more such stages.

The apparatus of the present invention preferably comprises (as in Fig. 1):
- 1: distillation column
- 2: first condenser

The distillation column may further contain:
- 3: feed (F) inlet
- 4: reflux (R) inlet
- 5: phenol product (P) outlet
- 6: base product outlet
- 7: column overhead outlet
- 8: reboiler

According to a preferred embodiment of the invention, the apparatus further comprises:
- 9: secondary condenser
- 10: condensate drum
- 11: vacuum unit
- 12: first condensate removal line
- 13: first uncondensed fraction connection line
- 14: light condensate reflux (LCR) line
- 15: light condensate removal line
- 16: second uncondensed fraction removal line

Thus, the apparatus used in the process of the present invention preferably comprises a distillation column 1 for the fractional distillation of a phenolic mixture, the column 1 containing a feed inlet 3, a reflux inlet 4, a phenol product outlet 5, a base product outlet 6 and a column overhead outlet 7. The feed inlet 3 is preferably positioned at the middle section or below the middle section of the column. The product outlet 5 is preferably positioned a few trays below the top tray of the column.

A portion of the base product removed from the column 1 through the base product outlet 6 may be returned to the column 1 through a reboiler 8.

At least one first condenser 2 is connected to the column 1 through the column overhead outlet 7 and the reflux inlet 4. The first condenser 2 is capable of separating the stream conducted to the condenser 2 from the overhead outlet 7 into a first condensate and a first uncondensed fraction. These separate in drum 10. The first condensate may then be conducted back to the distillation column 1 through the reflux inlet 4.

The first condensate and the first uncondensed fraction that are formed in the first condenser 2 may be conducted to a condensate drum 10, which is in fluid connection with the first condenser 2 and the reflux inlet 4 of the distillation column 1, and wherein the mentioned fractions may be separated. A portion of the first condensate is conducted to the distillation column 1 through the reflux inlet 4, while another portion of it may be removed through a first condensate removal line 12. Further, the first uncondensed fraction is conducted through a first uncondensed fraction connection line 13 to at least one secondary condenser 9. In the at least one secondary condenser 9 the first uncondensed fraction may be separated into a second condensate, a portion of which may be recycled back to the condensate drum 10 through a light condensate reflux line 14, while a second portion is removed through a light condensate removal line 15, and a second uncondensed fraction, which may be removed using a vacuum unit 11, such as a vacuum pump 11, through a second uncondensed fraction removal line 16.

The process and the apparatus may be used for removing by-products and impurities from any mixture containing at least two organic compounds. Preferably, the apparatus is arranged in the distillation section of a phenol production process. The phenol production process typically comprises process steps, wherein phenol and acetone are produced through the oxidation of cumene to cumene hydroperoxide (CHP) and, subsequently, wherein the CHP is concentrated and cleaved into phenol, acetone and other cleavage products, which products are washed and desalted, and finally wherein the desalted cleavage product mixture, containing, for example, phenol, acetone, water, cumene, AMS, hydroxyacetone, mesityl oxide, acetophenone, carbinol and heavy hydrocarbons, is distilled.

In the context of the present invention, the term "phenolic mixture" or "phenolic feed" refers to any product mixture or feed containing phenol as well as by-products and impurities at any stage of the purification process of the invention. The term "catalyst treatment feed" refers to the phenolic mixture being conducted to a catalytic purification reactor. Likewise, the term "distillation feed" refers to the phenolic feed being conducted to a distillation column.

As described above, at least a portion of the condensate(s) is recycled as reflux to the distillation of the present invention. The reflux stream ratio of condensate to product stream (R:P) is from 0.99:1 to 3:1, more preferably from 0.99:1 to 2:1, most preferably from 1.01:1 to 1.3:1. The reflux ratio of light condensate to product stream (LCR:P) is from 0:1 to 0.3:1, more preferably from 0:1 to 0.10:1 and most preferably from 0:1 to 0.03:1

The total amount of removed first and light condensate depends on the content of impurities and water in the distillation feed. A typical first condensate fraction removed from the product is from 0 % to 5 %, more preferably from 0 % to 2%, especially 1 to 2 wt%. A typical light condensate fraction removed from the product is from 0.01 % to 3 %, more preferably from 0.1 % to 2 %.

The amount of noncondensables conducted to the vacuum unit 11 depends on the amount of inert components and on the air leakage to the distillation.

The phenolic feed preferably contains at least 98.0 w-%, more preferably at least 99.0 w-%, and most preferably at least 99.7 w-% of phenol on a dry basis. Many impurities are present in the feed, including alpha-methylstyrene (AMS), acetol (hydroxyacetone), acetophenone residues, cyclohexanol, cumylphenols, 2-methylbenzofuran (2-MBF), phenyl dimethyl carbinol and other organic components, e.g. carbonyls, such as ketones, and dimers. The water content of the feed is smaller than 10,000 w-ppm, preferably smaller than 2000 w-ppm, more preferably smaller than 1000 w-ppm.

According to one embodiment of the present invention, the process may be operated also using water contents that are higher than 10,000 w-ppm, for example to shorten up the start-up process.

According to the present invention, the phenolic feed, i.e., the distillation feed, containing phenol and further by-products and impurities, as well as water, is conducted to the phenol distillation column, wherein it is distilled in order to separate the feed into a base product, an intermediate product and an overhead stream, whereby the base product contains most of the high boiling by-products and impurities, the overhead stream contains most of the low boiling by-products and impurities, such as cyclohexanol, and of the water or water-azeotropes as well as some phenol, and the intermediate product contains most of the product phenol. High boiling by-products and impurities are removed from the mixture as the base product through an outlet 6 at the base of the column, whereas water is removed, together with any low boiling compounds, with the overhead stream. The purified phenol product is removed as the intermediate product through an outlet 5 at an intermediate section of the column.

In the apparatus of the present invention, the overhead stream may be further separated into a first condensate and a first uncondensed fraction, the first condensate containing the higher boiling portion of the by-products and impurities, which are removed with a fraction of first condensate through the first condensate removal line 12.

According to a preferred embodiment of the present invention, the first uncondensed fraction may be again further separated into a light condensate and a second uncondensed fraction, the light condensate containing mainly phenol and water and low-boiling by-products and impurities, which are removed with a fraction of light condensate through the light condensate removal line 15, as well as the water and any components that may have formed azeotropes with water.

Using the process and the apparatus of the present invention, at least a portion of any by-products and impurities may be removed from a phenolic mixture, the portion consisting of 50-99% of the total amount of by-products and impurities in the distillation feed, preferably 60-99%, more preferably 70-99% on a dry basis.

The purification process and the apparatus of the present invention may be used to provide a pure phenol product with a "sufficiently low" water-content, which is intended to mean a water-content of below 500 ppm, preferably below 300 ppm, more preferably below 150 ppm, e.g. below 100 ppm, and most preferably below 60 ppm.

The distillation is carried out under low pressure, i.e., at a pressure of 20-300 kPa, preferably at a pressure below atmospheric pressure, more preferably at about 40-70 kPa, so that the thermal decomposition of the impurities is minimized. Decomposition would result in low boiling compounds that would be difficult to separate from the phenol product and would, therefore, cause fouling of the product.

The temperature in which the first condenser is operated is preferably about 80-170°C, preferably 110-150°C. The temperature in which the secondary condenser is operated is maintained above the solidification point of phenol, i.e. above 41°C, preferably between 42°C and 80°C.

The fractional distillation column 1 is divided into three zones, a stripping zone with a purpose to concentrate high boiling impurities to the base of the column 1, an intermediate zone used for the separation of high boiling impurities from a phenolic feed, and a pasteurization zone with the purpose to separate water and possible lighter hydrocarbons from the phenolic feed. The purified phenol product is removed through an outlet at the bottom of the pasteurization zone.

Further, the apparatus of the present invention comprises at least one condenser 2.

According to a preferred embodiment of the present invention, the apparatus of the present invention comprises at least two condensers 2, 9, which are arranged in series, thereby allowing low boiling by-products and impurities as well as water azeotropes to be concentrated in the mentioned light condensate. The light condensate is either removed or a portion of it is returned as reflux to the fractional distillation column 1 through the reflux inlet 4 via the condensate drum 10. Ideally, a portion is recycled, e.g. up to 10 wt% of the second condensate.

An advantage provided by the present invention is thus that high quality phenol can be produced by removing the light condensate. The total sum of removed first condensate and light condensate can be significantly smaller compared to an arrangement where only the first condensate is removed. In some applications all of the first condensate is returned to the column 1 as reflux.

The reflux fraction returned to the column 1 is conducted through the reflux inlet 4, which is positioned in the pasteurization zone of the column 1. The purpose of the reflux is to flush a portion of water and possible lighter hydrocarbons from the distillation column 1 and, thus, provide a more efficient recovery of phenol. This portion of water and light hydrocarbons is removed through the first uncondensed fraction, which preferably contains these components at a higher concentration than the first condensate. The removed portion containing by-products and impurities may then be conducted to a second condenser 9 to produce a second condensate and, thus, ensure liquification of the components of this fraction at a lower temperature than in other applications. The second condensate thus contains a concentrated amount of by-products and impurities as well as water.

The phenol product water content is thus controlled using the mentioned first condensate and light condensate. Likewise is the concentration of heavy by-products and impurities controlled by the bottom stream. This control is desirable in order to ensure a low concentration of impurities in the base of the column 1, because thermal decomposition is to be avoided. A maximum level is about 0.1-5 weight-%.

With the process of the present invention, the water concentration of the final purified phenol product is lowered compared to prior art, as is the concentration of by-products and impurities, such as 2-methyl benzofuran, hydroxyaetone, AMS and some ketones. This is achieved by controlling the amount of condensate reflux in the column 1 as in the present invention. As a result, more phenol is recovered in the purification column 1 of the present invention compared to the columns of conventional purification assemblies.

A portion of the by-products and impurities may be removed from the phenolic mixture through treatment with a catalyst in a catalytic purification reactor before conducting the mixture to the distillation column 1. This is achieved by contacting the phenolic mixture containing phenol, water and two or more further organic components, such as 2-methylbenzofuran, hydroxyacetone and carbonyls, including ketones and acids, with a catalyst, such as an acidic resin, whereby a portion of the by-products and impurities, i.e. a portion of the two or more organic components, are separated from the phenolic mixture or react to form higher boiling compounds and a phenolic feed containing phenol, water and at least one further organic component is formed. The catalyst causes at least a portion of the by-products (such as hydroxyacetone and carbonyls) in the catalyst treatment feed to react and form heavier compounds that can be separated from the phenol in the final distillation step.

The present invention is particularly advantageous in applications where the condenser(s) 2 (or 9) of the phenol purification column 1 is heat integrated. The condenser 2 may for example function as a reboiler for another column. In this case it is important to maintain a high and constant temperature in the condenser 2. The present invention allows the use of a higher temperature in the condenser 2 compared to prior art by recycling the light condensate to the condensate drum. Since the light condensate is more concentrated than the first one, removing the light condensate will allow the use of a flow of a smaller volume for removing the same amount of light components than when removing only the first condensate.

An advantage of the present invention is that it keeps the water concentration low in the column overhead and condensers. This decreases the risk of acid corrosion. Acids reaching the distillation of the present invention may stem, for example, from an upstream cleavage section or from a resin purification catalyst, if it is used prior to the distillation.

Typically, in prior art technology a vent gas condenser is used to recover phenol from the vent gases from the first condenser 2. This vent condensate is in prior art technology returned to an condensate drum of the column 1. Since a purpose of the reflux is to flush water and impurities out of the column 1, there will be a significantly increased need for condensing gases in the vent gas condenser and returning them through the condensate drum to the column 1 in cases where the water concentration of the phenolic feed to be purified is high. Water has only two outlets, i.e. it can be removed with the first condensate and, thus, in the above-mentioned case, the water concentration will increase, or it can be removed with the uncondensed fraction, the amount of which depends on the condensing temperature and the operating pressure. The water concentration will thus be limited by the separation efficiency of the column, the vapor-liquid equilibrium and the amount of removed condensate. The capacity of the vent gas condenser is small compared to the main condenser. When the cooling capacity is exceeded, the volumetric flow to the vacuum unit increases and this may cause process upset. The present invention thus solves the vent gas condenser capacity problem by removing a small concentrated second condensate stream.

According to a particular embodiment of the present invention, a small volume, i.e. less than 10,000 w-ppm, preferably less than 2000 w-ppm, most preferably less than 1000 w-ppm, of water is intentionally injected into the phenolic feed to improve the separation of impurities in the distillation column 1.

### Examples

In order to show the sensitivity of the removed light condensate stream vs the removed first condensate stream, three phenol purification column simulations were performed. The simulation model had water as a light component. The column operating conditions are summarized below in Table 1, Table 2 and Table 3. The feed water concentration (3000 ppm), the feed rate (10000 kg/h), the bottom rate and the reboiler duty were the same in all examples. However, the reflux rates (R/P, LCR/P) and the amounts of removed condensate were changed in the simulations. The first condenser temperature was fixed to 166°C and the secondary condenser temperature was fixed to 50°C.

### Example 1

In the first simulation (Table 1) only 132 kg/h of light condensate was removed from the circulation. The first condensate was not removed. The amount of product was 9587 kg/h. This gave a final product with a water concentration of 80 w-ppm. The first condenser duty was 2700 kW and the secondary condenser duty was 119 kW.

### Example 2

In the second simulation (Table 2) 130 kg/h of first condensate was removed. The light condensate from the secondary condenser was returned as reflux through the condensate drum to the distillation. The light condensate was not removed. From Tables 1 and 2 it can be seen that the condensing duty in the secondary condenser has increased from 119 to 2851 kW. The first condenser duty is zero. However, condensing occurs because 4699 kg/h of subcooled (50°C) light condensate reflux is mixed in the condensate drum with the overhead stream. Because of this, the condensing reflux to the column is 10844 kg/h. This gives a product with a water concentration as high as 370 w-ppm, which is almost three times the water concentration of example 1. This example shows also how efficient the light condensate purge is in controlling water build-up in condensers. The first condenser has lost its condensing capacity and a lower temperature would be needed for condensing.

### Example 3

In the third simulation no light condensate was removed. The amount of removed first condensate was increased to 800 kg/h to decrease the water concentration in the product. This gave a product with a water concentration as high as 110 ppm. However, water accumulation took place and the secondary condenser duty was 779 kW. Phenol production dropped in this case to 8917 kg/h.

| Table 1. Simulation 1: Only light condensate removed | | | | | | |
|---|---|---|---|---|---|---|
| | Feed | Bottom | Product | Condensate | Light condensate | To vacuum |
| stream ref. to figure 1 | 3 | 6 | 5 | 12 | 15 | 16 |
| Water | 0,300 % | 0,000 % | 0,008 % | | 20,594 % | 16,694 % |
| Phenol | 99,550 % | 94,627 % | 99,992 % | | 79,369 % | 1,634 % |
| Heavies | 0,150% | 5,373 % | 0,000 % | | 0,000 % | 0,000 % |
| Air | 0% | 0% | 0% | | 0,037 % | 81,673 % |
| Total Flow kg/hr | 10000 | 279 | 9587 | 0 | 132 | 12 |
| Temperature C | 193 | 166 | 158 | | 50,0 | 50,0 |
| Pressure bar | 9 | 0,62 | 0,50 | | 0,45 | 0,45 |
| | duty | temperature | | | | |
| | kW | C | | | | |
| Reboiler | 2600 | 166 | | R/P | 1.81 | |
| Condenser | 2730 | 133 | | LCR/P | 0.028 | |
| Secondary condenser | 119 | 50 | | | | |

| Table 2. Simulation 2: No light condensate removed | | | | | | |
|---|---|---|---|---|---|---|
| | Feed | Bottom | Product | Condensate | Light condensate | To vacuum |
| stream ref. to figure 1 | 3 | 6 | 5 | 12 | 15 | 16 |
| Water | 0,300 % | 0,000 % | 0,037 % | 18,523 % | | 18,746 % |
| Phenol | 99,550 % | 94,667 % | 99,963 % | 81,477 % | | 1,189 % |
| Heavies | 0,150 % | 5,333 % | 0,000 % | 0,000 % | | 0,000 % |
| Air | 0% | 0 % | 0% | 0% | | 80,065 % |
| Total Flow kg/hr | 10000 | 281 | 9587 | 130 | 0 | 12 |
| Temperature C | 193 | 166 | 157 | 82 | | 50 |
| Pressure bar | 9 | 0,62 | 0,50 | 0,45 | | 0,45 |
| | duty | temperature | | | | |
| | kW | C | | | | |
| Reboiler | 2600 | 166 | | R/P | 1.13 | |
| Condenser | 0 | 133 | | LCR/P | 0.49 | |
| Secondary condenser | 2851 | 50 | | | | |

| Table 3. Simulation 2: No light condensate removed | | | | | | |
|---|---|---|---|---|---|---|
| | Feed | Bottom | Product | Condensate | Light condensate | To vacuum |
| stream ref. to figure 1 | 3 | 6 | 5 | 12 | 15 | 16 |
| Water | 0,30% | 0,00% | 0,011 % | 3,33 % | | 18,57 % |
| Phenol | 99,55 % | 94,66 % | 99,99 % | 96,67 % | | 1,31 % |
| Heavies | 0,150% | 5,338 % | 0,000 % | 0,000 % | | 0,000 % |
| Air | 0% | 0% | 0% | 0% | | 80,12 % |
| Total Flow kg/hr | 10000 | 281 | 8917 | 800 | 0 | 12 |
| Temperature C | 193 | 166 | 158 | 104 | | 50 |
| Pressure bar | 9 | 0,62 | 0,50 | 0,45 | | 0,45 |
| | duty | temperature | | | | |
| | kW | C | | | | |
| Reboiler | 2600 | 166 | | R/P | 1.63 | |
| Condenser | 2087 | 133 | | LCR/P | 0.18 | |
| Secondary condenser | 779 | 50 | | | | |

## Claims

1. A process for purifying a phenolic mixture, which process comprises
- as a first step, providing a phenolic feed containing phenol, water and at least one further organic or acidic component, and
- as a second step, separating the phenolic feed by distillation into a base fraction, an intermediate fraction and a first overhead stream,
- conducting the first overhead stream through at least one condenser to an condensate drum 10 in which at least one first condensate and at least one first uncondensed fraction are formed,
- recycling at least a portion of the first condensate to the distillation;
- conducting the first uncondensed fraction to a second condenser wherein a second condensed fraction and a second uncondensed fraction are formed;
- removing at least a portion of the second uncondensed fraction;
- recycling at least a portion of the second condensed fraction to the condensate drum 10,
- removing at least a portion of said first and/or second condensed fraction.

2. The process of claim 1, **characterized by** separating the first overhead stream, which contains phenol, water and low-boiling components or water azeotropes, into at least one first condensate, which contains mainly phenol, and at least one first uncondensed fraction, which contains mainly phenol, water and optional low-boiling components and noncondensables.

3. The process of claim 1 or 2, **wherein** the intermediate fraction is recovered as the phenol product fraction.

4. The process of claim 1 to 3, **characterized by** removing the base fraction containing high boiling impurities, such as cumyl phenols and acetophenone.

5. The process of claim 1 to 4, **characterized by** carrying out the distillation at a pressure of 20-300 kPa, preferably at a pressure below atmospheric pressure, more preferably at 40-70 kPa.

6. The process of any preceding claim, **characterized by** conducting the first uncondensed fraction(s) through at least one secondary condenser, wherein the fraction(s) is at least partially condensed to produce at least one second condensate (light condensate), containing mainly phenol and water and low-boiling components or water azeotropes, and a second uncondensed fraction, and removing at least a portion of the second condensate.

7. The process of any preceding claim, **characterized by** operating the secondary condenser(s) at a temperature above the freezing point of phenol, i.e. at above 41 °C.

8. The process of any preceding claim, **characterized by** providing a phenolic mixture containing phenol, water and two or more further organic components, such as 2-methylbenzofuran, hydroxyacetone and carbonyls, including ketones and acids.

9. The process of claim 8, **characterized by** conducting the phenolic mixture containing phenol, water and two or more further organic components through a catalyst treatment to separate a portion of the two or more organic components from the mixture or to cause reactions, thus forming higher boiling compounds from the two or more organic compounds, in order to produce the phenolic feed containing phenol, water and at least one further organic component, which is provided in the first step of claim 1.

10. The process of any preceding claim, **characterized by** injecting water into the phenolic feed containing phenol, water and at least one further organic component.

11. The process of any preceding claim wherein all the first condensate is recycled or wherein at least a portion of the first condensate is removed and a portion of the second condensate is removed.

12. The process of any preceding claim wherein at least a portion of the second condensate is recycled.

13. An apparatus for purifying a phenolic mixture, comprising a distillation column (1) containing a feed inlet (3), a recycle inlet (4), a phenol product outlet (5), a base product outlet (6) and a distillate outlet (7),
at least one partial condenser (2) connected to the distillate outlet (7) and to condensate drum 10, a recycle inlet (4) connected to condensate drum (4) and an outlet 12 for removing condensate from drum 10, a second partial condenser (9) arranged in series with condenser (2), said condenser (9) being provided with a line (14) arranged to recycle material from second condenser (9) to drum 10, and a line 15 arranged to remove condensate from condenser 9) wherein the the phenol distillation column (1) is preferably divided into three zones, a stripping zone with a purpose to concentrate high boiling components to the base of the column (1), an intermediate zone meant for the separation of high boiling components from the phenol product, and a pasteurization zone with the purpose to separate water and possible lighter hydrocarbon components from the phenol product.

14. The apparatus of claim 13, **characterized in** further comprising a catalytic treatment reactor comprising a catalyst, preferably a resin, connected to the feed inlet (3) of the distillation column (1).

15. The use of the apparatus or process of any of claims 1 to 14 for removing by-products and impurities from any mixture containing at least two organic compounds.

## Patentansprüche

1. Verfahren zum Reinigen einer phenolischen Mischung, wobei das Verfahren das Folgende umfasst:
- als einen ersten Schritt, Bereitstellen einer phenolischen Zufuhr, die Phenol, Wasser und mindestens einen weiteren organischen oder sauren Bestandteil enthält, und
- als einen zweiten Schritt, Trennen der phenolischen Zufuhr mittels Destillation in eine Basisfraktion, eine Zwischenfraktion und einen ersten Kopfproduktstrom,
- Leiten des ersten Kopfproduktstroms durch mindestens einen Kondensator zu einer Kondensattrommel 10, in der mindestens ein erstes Kondensat und mindestens eine erste unkondensierte Fraktion gebildet werden,
- Recyceln von mindestens einem Teil des ersten Kondensats zu der Destillation;
- Leiten der ersten unkondensierten Fraktion zu einem zweiten Kondensator, wobei eine zweite kondensierte Fraktion und eine zweite unkondensierte Fraktion gebildet werden;
- Entfernen von mindestens einem Teil der zweiten unkondensierten Fraktion;
- Recyceln von mindestens einem Teil der zweiten kondensierten Fraktion zu der Kondensattrommel 10,
- Entfernen von mindestens einem Teil von der ersten und/oder zweiten kondensierten Fraktion.

2. Verfahren nach Anspruch 1, **gekennzeichnet durch** das Trennen des ersten Kopfproduktstroms, der Phenol, Wasser und niedersiedende Bestandteile oder Wasserazeotrope enthält, in mindestens ein erstes Kondensat, das hauptsächlich Phenol enthält, und mindestens eine zweite unkondensierte Fraktion, die hauptsächlich Phenol, Wasser und optional niedersiedende Bestandteile und nicht kondensierbare Bestandteile enthält.

3. Verfahren nach Anspruch 1 oder 2, wobei die Zwischenfraktion als die Phenolproduktfraktion gewonnen wird.

4. Verfahren nach Anspruch 1 bis 3, **gekennzeichnet durch** das Entfernen der Basisfraktion, die hochsiedende Verunreinigungen enthält, wie Cumylphenole und Acetophenon.

5. Verfahren nach Anspruch 1 bis 4, **gekennzeichnet durch** das Ausführen der Destillation bei einem Druck von 20 - 300 kPa, vorzugsweise bei einem Druck unter Atmosphärendruck, besonders vorzugsweise bei 40 - 70 kPa.

6. Verfahren nach einem der vorhergehenden Ansprüche, **gekennzeichnet durch** das Leiten der ersten unkondensierten Fraktion(en) **durch** mindestens einen zweiten Kondensator, wobei die Fraktion(en) zumindest teilweise kondensiert wird, um mindestens ein zweites Kondensat (leichtes Kondensat), das hauptsächlich Phenol, Wasser und niedersiedende Bestandteile oder Wasserazeotrope enthält, und eine zweite unkondensierte Fraktion zu erzeugen, und das Entfernen von mindestens einem Teil des zweiten Kondensats.

7. Verfahren nach einem der vorhergehenden Ansprüche, **gekennzeichnet durch** das Betreiben des(r) zweiten Kondensators(en) bei einer Temperatur über dem Gefrierpunkt von Phenol, d.h. über 41 °C.

8. Verfahren nach einem der vorhergehenden Ansprüche, **gekennzeichnet durch** das Bereitstellen einer phenolischen Mischung, die Phenol, Wasser und zwei oder mehr weitere organische Bestandteile enthält, wie zum Beispiel 2-Methylbenzofuran, Hydroxyaceton und Carbonyle, einschließlich von Ketonen und Säuren.

9. Verfahren nach Anspruch 8, **gekennzeichnet durch** das Leiten der phenolischen Mischung, die Phenol, Wasser und zwei oder mehr weitere organische Bestandteile enthält, **durch** eine Katalysatorbehandlung, um einen Teil von den zwei oder mehr organischen Bestandteilen von der Mischung zu trennen oder um Reaktionen hervorzurufen, wodurch höhersiedende Verbindungen aus den zwei oder mehr organischen Verbindungen gebildet werden, um die phenolische Zufuhr, die Phenol, Wasser und mindestens einen weiteren organischen Bestandteil enthält, zu erzeugen, die in dem ersten Schritt von Anspruch 1 bereitgestellt wird.

10. Verfahren nach einem der vorhergehenden Ansprüche, **gekennzeichnet durch** das Einspeisen von Wasser in die phenolische Zufuhr, die Phenol, Wasser und mindestens einen weiteren organischen Bestandteil enthält.

11. Verfahren nach einem der vorhergehenden Ansprüche, wobei das gesamte erste Kondensat recycelt wird oder wobei mindestens ein Teil von dem ersten Kondensat und ein Teil von dem zweiten Kondensat entfernt werden.

12. Verfahren nach einem der vorhergehenden Ansprüche, wobei mindestens ein Teil von dem zweiten Kondensat recycelt wird.

13. Apparat zum Reinigen einer phenolischen Mischung, umfassend eine Destillationskolonne (1), die einen Zufuhreinlass (3), einen Recyclingeinlass (4), einen Phenolproduktauslass (5), einen Basisproduktauslass (6) und einen Destillatauslass (7) enthält,
mindestens einen teilweisen Kondensator (2), der mit dem Destillatauslass (7) und der Kondensattrommel 10 verbunden ist, einen Recyclingeinlass (4), der mit der Kondensattrommel (4) und einem Auslass 12 zum Entfernen des Kondensats aus der Trommel 10 verbunden ist, einen zweiten teilweisen Kondensator (9), der mit dem Kondensator (2) in Reihe angeordnet ist, wobei der Kondensator (9) mit einer Leitung (14) ausgestattet ist, die so angeordnet ist, dass sie das Material aus dem zweiten Kondensator (9) zur Trommel 10 recycelt, und eine Leitung 15, die so angeordnet ist, dass sie Kondensat aus dem Kondensator (9) entfernt, wobei die Phenoldestillationskolonne (1) vorzugsweise in drei Zonen unterteilt ist, eine Strippzone mit einem Zweck, hochsiedende Bestandteile an der Basis der Kolonne (1) zu konzentrieren, eine Zwischenzone, die für die Trennung der hochsiedenden Bestandteile von dem Phenolprodukt gedacht ist, und eine Pasteurisierungszone mit dem Zweck, Wasser und mögliche leichtere Kohlenwasserstoffbestandteile von dem Phenolprodukt zu trennen.

14. Apparat nach Anspruch 13, ferner **dadurch gekennzeichnet, dass** er einen katalytischen Behandlungsreaktor umfasst, der einen Katalysator, vorzugsweise ein Harz, umfasst und der mit dem Zufuhreinlass (3) der Destillationskolonne (1) verbunden ist.

15. Verwendung des Apparats oder Verfahrens nach einem der Ansprüche 1 14 zum Entfernen von Nebenprodukten und Verunreinigungen aus einer Mischung, die mindestens zwei organische Verbindungen enthält.

## Revendications

1. Procédé de purification d'un mélange phénolique, lequel procédé comprend
- en tant que première étape, la fourniture d'une alimentation phénolique contenant du phénol, de l'eau et au moins un autre composant organique ou acide, et
- en tant que seconde étape, la séparation de l'alimentation phénolique par distillation en une fraction de base, une fraction intermédiaire et un premier courant de tête,
- l'envoi du premier courant de tête à travers au moins un réfrigérant vers un tambour de condensat 10 dans lequel au moins un premier condensat et au moins une première fraction non condensée sont formés,
- le recyclage d'au moins une partie du premier condensat dans la distillation ;
- l'envoi de la première fraction non condensée vers un second réfrigérant dans lequel une seconde fraction condensée et une seconde fraction non condensée sont formées ;
- l'élimination d'au moins une partie de la seconde fraction non condensée ;
- le recyclage d'au moins une partie de la seconde fraction condensée dans le tambour de condensat 10,
- l'élimination d'au moins une partie de ladite première et/ou de ladite seconde fraction condensée.

2. Procédé selon la revendication 1, **caractérisé par** la séparation du premier courant de tête, qui contient du phénol, de l'eau et des composants à bas point d'ébullition ou des azéotropes avec l'eau, en au moins un premier condensat, qui contient principalement du phénol, et au moins une première fraction non condensée, qui contient principalement du phénol, de l'eau et des composants à bas point d'ébullition facultatifs et des composés non condensables.

3. Procédé selon la revendication 1 ou 2, dans lequel la fraction intermédiaire est récupérée en tant que fraction de phénol produit.

4. Procédé selon la revendication 1 à 3, **caractérisé par** l'élimination de la fraction de base contenant des impuretés à point d'ébullition élevé, tels que les cumylphénols et l'acétophénone.

5. Procédé selon la revendication 1 à 4, **caractérisé par** la réalisation de la distillation à une pression de 20 à 300 kPa, de préférence à une pression inférieure à la pression atmosphérique, de manière davantage préférée de 40 à 70 kPa.

6. Procédé selon une quelconque revendication précédente, **caractérisé par** l'envoi de la ou des premières fractions non condensées à travers au moins un réfrigérant secondaire, dans lequel la ou les fractions sont au moins partiellement condensées pour produire au moins un second condensat (condensat léger), contenant principalement du phénol et de l'eau et des composants à bas point d'ébullition ou des azéotropes avec l'eau, et une seconde fraction non condensée, et l'élimination d'au moins une partie du second condensat.

7. Procédé selon une quelconque revendication précédente, **caractérisé par** le fonctionnement du ou des réfrigérants secondaires à une température au-dessus du point de congélation du phénol, à savoir au-dessus de 41 °C.

8. Procédé selon une quelconque revendication précédente, **caractérisé par** la fourniture d'un mélange phénolique contenant du phénol, de l'eau et deux ou plusieurs autres composants organiques, tels que le 2-méthylbenzofurane, l'hydroxyacétone et des composés carbonyle, y compris des cétones et des acides.

9. Procédé selon la revendication 8, **caractérisé par** l'envoi du mélange phénolique contenant du phénol, de l'eau et deux ou plusieurs autres composants organiques par un traitement avec un catalyseur pour séparer une partie des deux ou plusieurs composants organiques du mélange ou pour provoquer des réactions, formant ainsi des composés à point d'ébullition plus élevé à partir des deux ou plusieurs composés organiques, afin de produire l'alimentation phénolique contenant du phénol, de l'eau et au moins un autre composant organique, qui est fourni dans la première étape de la revendication 1.

10. Procédé selon une quelconque revendication précédente, **caractérisé par** l'injection d'eau dans l'alimentation phénolique contenant du phénol, de l'eau et au moins un autre composant organique.

11. Procédé selon une quelconque revendication précédente, dans lequel la totalité du premier condensat est recyclée ou dans lequel au moins une partie du premier condensat est éliminée et une partie du second condensat est éliminée.

12. Procédé selon une quelconque revendication précédente, dans lequel au moins une partie du second condensat est recyclée.

13. Appareil de purification d'un mélange phénolique, comprenant une colonne de distillation (1) contenant une entrée d'alimentation (3), une entrée de recyclage (4), une sortie de phénol produit (5), une sortie de produit de base (6) et une sortie de distillat (7),
au moins un réfrigérant partiel (2) relié à la sortie de distillat (7) et au tambour de condensat 10, une entrée de recyclage (4) reliée au tambour de condensat (4) et une sortie 12 pour éliminer le condensat du tambour 10, un second réfrigérant partiel (9) disposé en série avec le réfrigérant (2), ledit réfrigérant (9) étant fourni avec une ligne (14) disposée pour recycler de la matière recyclée provenant du second réfrigérant (9) vers le tambour 10, et une ligne 15 disposée pour éliminer le condensat provenant du réfrigérant (9) dans lequel la colonne de distillation de phénol (1) est de préférence divisée en trois zones, une zone de décapage dans le but de concentrer les composants à point d'ébullition élevé à la base de la colonne (1), une zone intermédiaire pour la séparation des composants à point d'ébullition élevé du phénol produit, et une zone de pasteurisation dans le but de séparer l'eau et les composants hydrocarbonés plus légers éventuels du phénol produit.

14. Appareil selon la revendication 13, **caractérisé en ce qu'**il comprend en outre un réacteur de traitement catalytique comprenant un catalyseur, de préférence une résine, relié à l'entrée d'alimentation (3) de la colonne de distillation (1).

15. Utilisation de l'appareil ou du procédé selon l'une quelconque des revendications 1 à 14 pour éliminer des sous-produits et des impuretés d'un mélange quelconque contenant au moins deux composés organiques.
